# EUROPÄISCHE PATENTSCHRIFT

(11) **EP 1 034 797 B1**
(45) Veröffentlichungstag und Bekanntmachung des Hinweises auf die Patenterteilung: **28.04.2004**
(21) Anmeldenummer: 00103937.9
(22) Anmeldetag: 25.02.2000
(51) Int. Cl.: A61K 51/04

(54) **Verfahren zur Herstellung eines 18 F-markierten Glykosylierungsreagenz sowie 18 F-Glykosylierungsreagenz und dessen Verwendung**
Process for the manufacture of an 18F-labelled glycosylation reagent as well as 18F-glycosylation reagent and its use
Procédé d'obtention d'un réactif de glycosylation marqué au fluor-18 ainsi qu'un réactif de glycosylation marqué au fluor-18 et son utilisation

(30) Priorität: 01.03.1999 DE 19908712
(43) Veröffentlichungstag der Anmeldung: 13.09.2000
(73) Patentinhaber: Forschungszentrum Jülich GmbH, 52425 Jülich (DE)
(72) Erfinder: Prante, Olaf, 91052 Erlangen (DE); Hamacher, Kurt, Dr., 52076 Aachen (DE); Coenen, Heinz H., Prof., 41516 Grevenbroich (DE)

(56) Entgegenhaltungen:
- EP-A- 0 167 103
- WO-A-98/25940
- WO-A-98/33894
- JOURNAL OF LABELLED COMPOUNDS AND RADIOPHARMACEUTICALS, Bd. 42, Nr. Suppl. 1, Juni 1999 (1999-06), Seiten S111-S112, XP000916667
- HAAPARANTA M ET AL: "Pharmacokinetics and Metabolism of 2-[F]Fluoro-2-deoxy-d-glucose (FDG) in Mammary Tumors of Antiestrogen-treated Rats" NUCLEAR MEDICINE AND BIOLOGY,US,ELSEVIER SCIENCE PUBLISHERS, NEW YORK, NY, Bd. 22, Nr. 4, 1. Mai 1995 (1995-05-01), Seiten 483-489, XP004051779 ISSN: 0969-8051

## Beschreibung

Die Erfindung betrifft ein Verfahren zur Herstellung eines ¹⁸F-markierten Glykosylierungsreagenz sowie ein Glykosylierungsreagenz und dessen Verwendung.

Die zunehmende Bedeutung ¹⁸F-markierter Verbindungen in der nuklearmedizinischen Funktionsdiagnostik sowie das Interesse, die pharmakokinetischen Eigenschaften von Biopolymeren nicht invasiv mittels Positronen-Emissions-Tomographie (PET) zu untersuchen, hat dazu geführt, das Spektrum der selektiven Markierungsmethoden zu erweitern. So besteht Interesse an ¹⁸F-markierten Glykosylierungsreagenzien, die zur selektiven Markierung glykosylierter Peptide und Proteine sowie von Oligosacchariden eingesetzt werden können. Solche mit ¹⁸F markierten Biopolymere können sowohl für die in vivo pharmakokinetische Bewertung als auch für diagnostische Zwecke mittels der Positronen-Emissions-Tomographie eingesetzt werden. Da diese Biopolymere im lebenden Körper eingesetzt werden, dürfen die applizierten Stoffmengen keine toxikologische Wirkung aufweisen. Das zur Diagnostik notwendige ¹⁸F hat nur eine verhältnismäßig kurze Halbwertszeit von T_{1/2} = 110 min, so daß die tomographische Erfassung des Radioisotops nur in einem relativ kurzen Zeitfenster zur Verfügung steht, so daß die auftretende Strahlenbelastung als gering bewertet werden kann.

Es ist die Aufgabe der Erfindung ein neues ¹⁸F-Markierungsreagenz zur Verfügung zu stellen, das für die regioselektive ¹⁸F-Markierung von Biopolymeren geeignet ist. Weiterhin soll ein Herstellungsverfahren geschaffen werden, das eine schnelle Synthese des ¹⁸F-Markierungsreagenz ermöglicht, welche die Verfügbarkeit einer ausreichenden Aktivitätsmenge für die nachfolgende ¹⁸F-Glykosylierungsreaktion von Biopolymeren gewährleistet.

Ausgehend vom Oberbegriff des Anspruchs 1 wird die Aufgabe erfindungsgemäß gelöst mit den im kennzeichnenden Teil des Anspruchs 1 angegebenen Merkmalen.

Das neue Markierungsreagenz ermöglicht die Herstellung von ¹⁸F-markierten glykosylierten Peptiden und glykosylierten Proteinen sowie von ¹⁸F-markierten Polysacchariden und niedermolekularen bzw. Oligosacchariden. Das erfindungsgemäße Verfahren für die Herstellung des ¹⁸F-Markierungsreagenz ermöglicht kurze Synthesezeiten, mit der Folge einer längeren verbleibenden Nutzungszeit des Markierungsreagenz. Die Ausbeuten betragen 30 bis 35 %. Die erfindungsgemäßen Markierungsstoffe können trägerarm, das heißt weitestgehend ohne Anteile an ¹⁹F (trägerarm) zur Verfügung gestellt werden.

Die Figuren zeigen Formeln und Reaktionsgleichungen.

Es zeigt:
- Fig.1:: Eine Reaktionsgleichung
- Fig.2:: Strukturformel des erfindungsgemäßen Produkts
- Fig.3:: Reaktionsgleichungen für die Herstellung von ¹⁸F-markierten Biopolymeren.

Vorteilhafte Weiterbildungen der Erfindung sind in den Unteransprüchen angegeben.

Im folgenden soll die Erfindung erläutert werden.

Nach dem erfindungsgemäßen Verfahren wird eine Hexose der allgemeinen Formel 1 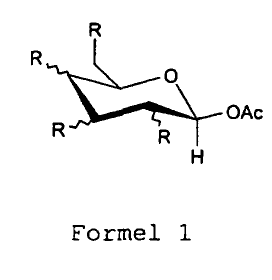 als Ausgangsstoff eingesetzt. Sie besitzt vier Reste R, welche in 2,3,4 und 6 Stellung positioniert sind. Von den vier Resten bedeuten drei eine Schutzgruppe, welche über ein Sauerstoffatom an den Sechsring gebunden ist. Der vierte Rest ist ein ¹⁸F-Atom, welches sich in einer der Positionen aus der Gruppe von 2,3,4, und 6 befindet. Als Schutzgruppen sind besonders gut OAc-Gruppen geeignet, jedoch können auch andere, der Hydrolyse zugängliche Schutzgruppen eingeführt sein. Ein bevorzugter Ausgangsstoff ist 1.3.4.6-Tetra-O-acetyl-2-[¹⁸F]fluor-2-desoxy-D-glucose, das das Vorprodukt für die Synthese von Fluordesoxy-D-Glucose ist.

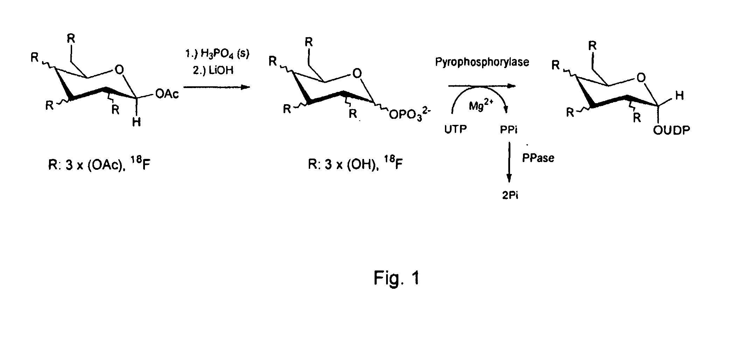

Erfindungsgemäß wird gemäß dem Reaktionsschema nach Figur 1 eine trägerarme Verbindung gemäß Formel 1 unter wasserfreien Bedingungen in einer Phosphorsäureschmelze zum Tri-O-acetyl[¹⁸F]fluordesoxy-D-glucose-1-phosphat umgesetzt, das im nächsten Reaktionsschritt unter basischen Bedingungen im wäßrigen System deacetyliert wird. Die bevorzugte Base ist LiOH, da der Phosphatrest durch LiOH nicht abgespalten wird. Weiterhin wird überflüssiges Phosphat durch Li⁺ präzipitiert. Das erfindungsgemäße Reaktionsprodukt Uridindiphosphat[¹⁸F]fluordesoxy-D-glucose kann in einer enzymatisch katalysierten Umsetzung von Uridintriphosphat mit dem [¹⁸F]Fluordesoxy-D-glucose-1-phosphat in Gegenwart von Uridindiphosphatglucosepyrophosphorylase synthetisiert werden. Als Cofaktor für die enzymatische Reaktion wird Mg²⁺ eingesetzt.

Als ¹⁸F-markierte, geschützte Zucker kommen neben der in 2-Position ¹⁸F-markierten tetraacetylierten Desoxyglucose die in 3,4 und 6-Position ¹⁸F-markierten tetraacetylierten Desoxyhexosen in Betracht. So können zum Beispiel 1,2,4,6-Tetra-O-acetyl-3-[¹⁸F]fluor-3-desoxy-D-glucose, 1,2,3,4-Tetra-O-acetyl-6-[¹⁸F]fluor-6-desoxy-D-glucose oder 1,2,3,6-Tetra-O-acetyl-4-[¹⁸F]fluor-4-desoxy-D-glucose als Ausgangsverbindungen eingesetzt werden.

Als Pyrophosphorylasen können grundsätzlich alle bekannten Uridindiphosphatglucosepyrophosphorylasen eingesetzt werden (EC 2.7.7.9). Die Pyrophosphorylasen aus Rinderleber und Bäckerhefe (Sigma, TypX) sind kommerziell erhältlich und daher besonders gut zugänglich.

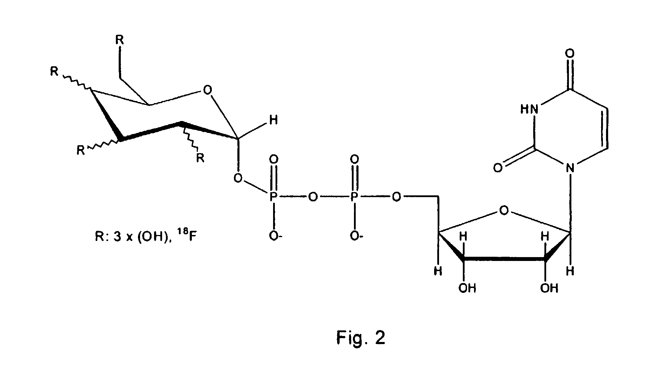

Die in Figur 2 angegebene Formel 2 bezeichnet die Struktur des erfindungsgemäßen ¹⁸F-Markierungsreagenz, welches vier Reste R besitzt.

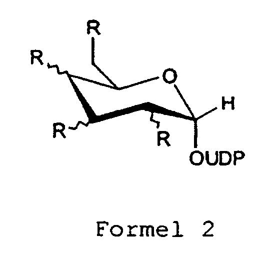

In der allgemeinen Formel 2 der Figur 2 befinden sich die Reste in den Positionen 2,3,4 und 6, wobei drei der Reste OH-Gruppen sind. Ein Rest ist ¹⁸F und kann sich in einer der Positionen aus der Gruppe von 2,3,4 und 6 befinden. Ein bevorzugtes Produkt ist Uridindiphosphat-2-[¹⁸F]Fluor-2-desoxy-D-glucose. Weitere Produkte sind: Uridindiphosphat-3-[¹⁸F]Fluor-3-desoxy-D-glucose, Uridindiphosphat-4-[¹⁸F]Fluor-4-desoxy-D-glucose und Uridindiphosphat-6-[¹⁸F]Fluor-6-desoxy-D-glucose.

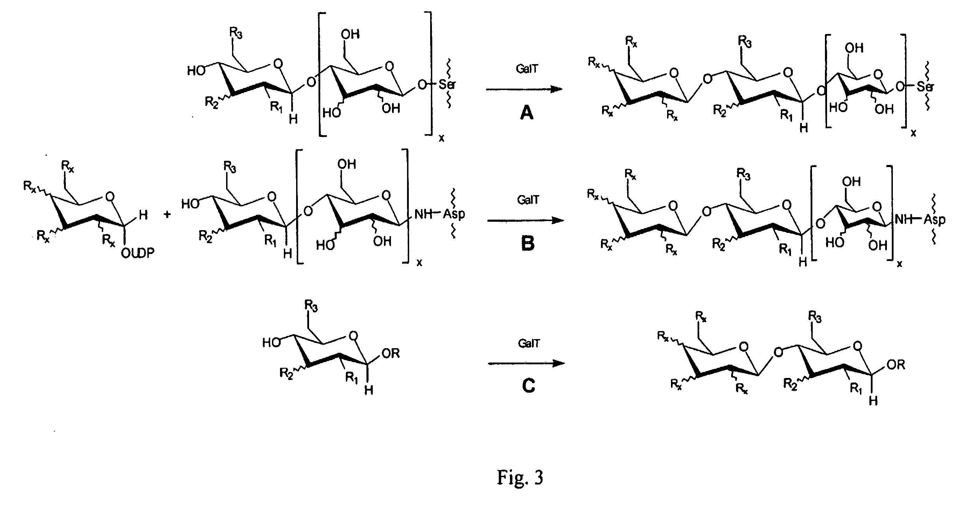

Die erfindungsgemäß synthetisierten Produkte gemäß Figur 2 können in enzymatischen Reaktionen unter Einsatz von Galactosyltransferasen mit O-Glykoproteinen, N-Glykoproteinen oder Oligosacchariden umgesetzt werden, deren Produkte als Biopolymere in der medizinischen Radiodiagnostik beziehungsweise Nuklearmedizin eingesetzt werden können.
Bei den in Figur 3 aufgeführten Reaktionen bedeuten:
A: ¹⁸F-Glykosylierung von O-Glycoproteinen,
B: ¹⁸F-Glykosylierung von N-Glycoproteinen und
C: ¹⁸F-Glykosylierung von Oligosacchariden.

Im erfindungsgemäßen Edukt sind vier Reste Rₓ in 2,3,4 und 6 Position von denen drei OH-Gruppen sind. Der vierte Rest Rₓ ist ein ¹⁸F-Atom, welches in einer der Positionen aus der Gruppe 2,3,4 und 6 angesiedelt sein kann.
In den O-Glykoproteinen, N-Glykoproteinen und Oligosacchariden bedeuten:
R: H, Oligosaccharylrest
R₁: NHAc, OH
R₂, R₃: H, OH, O-OH₃, S-CH₃, Fucosyl-
Die Abkürzung GalT bezeichnet eine Galactosyltransferase.

Mit dem erfindungsgemäßen Verfahren kann das ¹⁸F-markierte Coenzym Uridindiphosphat-[¹⁸F]fluordesoxy-D-glucose trägerarm synthetisiert werden. Das Herstellungsverfahren erlaubt die Synthese der erfindungsgemäßen Verbindung mit einer Synthesedauer, die zu einer guten radiochemischen Ausbeute des ¹⁸F-markierten Coenzyms führt, so daß der Einsatz für die enzymatische ¹⁸F-Glykosylierung von Glykoproteinen und Oligosacchariden realisiert werden kann.

### Ausführungsbeispiel:

1,3,4,6-Tetra-O-acetyl-2-[¹⁸F]fluor-2-desoxy-D-glucose wird getrocknet eingesetzt.
Die Vakuumschmelze mit kristalliner Phosphorsäure (10-20 mg) erfolgt bei der Reaktionstemperatur 70 °C und liefert eine radiochemische Ausbeute für 3,4,6-Tri-Oacetyl-2-[¹⁸F]fluor-2-desoxy-D-glucose-1-phosphat von 40-60% (bzgl. 1,3,4,6-Tetra-O-acetyl-2-[¹⁸F]fluordesoxy-D-glucose).

Die basische hydrolyse wird nach Aufnahme der Phosphorsäureschmelze in 0,2-0,3 ml Tetrahydrofuran mit 0,2 ml 2N Lithiumhydroxidlösung in der Kälte durchgeführt.

2-[¹⁸F]Fluor-2-desoxy-D-glucose-1-phosphat wird auf einem Anionenaustauscher (Accell QMA, Waters) fixiert und isoliert. Die eingesetzte Menge der festen Phase beträgt 50-100 mg.

Die enzymatische Umsetzung mit Uridindiphosphatglucosepyrophosphorylase verläuft im Zwischenkornvolumen der Polymermatrix, auf dem die phosphorylierte 2-[¹⁸F]Fluor-2-desoxy-D-glucose fixiert wird. Nach der Fixierung des trägerarmen 2-[¹⁸F]Fluor-2-desoxy-D-glucose-1-phosphat durch Ionenaustausch wird das Zwischenkornvolumen des Austauscherharzes (50...100µl) mit einem Substrat-Enzymcocktail gefüllt, der folgende Komponenten enthält: Uridintriphosphat, Uridindiphosphatglucosepyrophosphorylase, anorganische Pyrophosphatase, Mg²⁺ in Tris-Puffer. Die anionischen Bestandteile des Substrat-Enzymcocktails führen zu einer Desorption und homogenen Verteilung des 2-[¹⁸F]Fluor-2-desoxy-D-glucose-1-phosphat in der Flüssigkeitsphase des Zwischenkornvolumens.
Die Phosphorylase (E.C. 2.7.7.9) stammt aus Rinderleber und kann, ebenso wie die zur Beschleunigung der Reaktion eingesetzte anorganische Pyrophosphatase (E.C. 3.6.1.1), von Sigma-Aldrich bezogen werden.

Die Enzymkonzentrationen betragen für Uridindiphosphatglucosepyrophosphorylase 0,6 mg/ml und für anorganische Pyrophosphatase 0,03 mg/ml.

Die eingesetzte Magnesiumkonzentration beträgt 0,5 mM.

Die eingesetzte Substratkonzentration beträgt für Uridintriphosphat 0,5 mM und 2-[¹⁸F]Fluor-2-desoxy-D-glucose-1-phosphat wird in trägerarmer Form in submikromolaren Konzentrationen eingesetzt.

Der pH-Wert der wässrigen Lösung beträgt 8,0 (100 mM Tris-Puffer), die Reaktionstemperatur ist 40°C.

In dem kleinen Zwischenkornvolumen führt die Freisetzung des 2-[¹⁸F]Fluor-2-desoxy-D-glucose-1-phosphat in den an sich geringen Mengen zu einer hohen lokalen Konzentration, welche die Produktbildung beschleunigt. Daher wird die Reaktionszeit verkürzt und es verbleibt ein längerer Zeitraum für die Benutzung des kurzlebigen Produkts.
Die enzymatische Festphasenreaktion liefert nach einer Reaktionszeit von 60 Minuten eine radiochemische Ausbeute von 30% für trägerarmes Uridindiphosphat-2-[¹⁸F]fluor-2-desoxy-D-glucose. Die enzymatische Umsetzung kann durch Erhöhung der Enzymkonzentration beschleunigt werden.

## Patentansprüche

1. Verfahren zur Herstellung eines ¹⁸F-markierten Glykosylierungsagenz,
**dadurch gekennzeichnet,**
**daß** eine Hexose der allgemeinen Formel 1 mit 4 Resten R 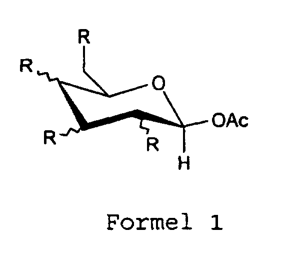
- wobei drei von den vier Resten eine Schutzgruppe bedeuten, die über Sauerstoffatome an den Sechsring gebunden sind,
- und ein Rest R ¹⁸F bedeutet, wobei sich R = ¹⁸F in der Position 2,3,4 oder 6 der Hexose befindet,
1. mit H₃PO₄ umgesetzt wird,
2. anschließend mit einer Base hydrolysiert wird,
3. und danach unter Zugabe von UTP mittels einer Pyrophosphorylase in Anwesenheit von Mg²⁺ umgesetzt wird.

2. Verfahren nach Anspruch 1,
**dadurch gekennzeichnet,**
**daß** eine Hexose der allgemeinen Formel 1 eingesetzt wird, deren Schutzgruppe R eine OAc-Gruppe ist.

3. Verfahren nach einem der Ansprüche 1 oder 2,
**dadurch gekennzeichnet,**
**daß** die Umsetzung gemäß Schritt 1. mit H₃PO₄ durch Verschmelzung erfolgt.

4. Verfahren nach einem der Ansprüche 1 bis 3,
**dadurch gekennzeichnet,**
**daß** die Hydrolyse mit einer Base gemäß Schritt 2. mittels LiOH erfolgt.

5. Verfahren nach einem der Ansprüche 1 bis 4,
**dadurch gekennzeichnet,**
**daß** in Schritt 3. eine Uridindiphosphatglucosepyrophosphorylase (EC 2.7.7.9) eingesetzt wird.

6. Verfahren nach Anspruch 6,
**dadurch gekennzeichnet,**
**daß** eine Uridindiphosphatglucosepyrophosphorylase aus Rinderleber oder Bäckerhefe eingesetzt wird.

7. Verfahren nach einem der Ansprüche 1 bis 6,
**dadurch gekennzeichnet,**
**daß** als Verbindung gemäß Formel 1 1.3.4.6-Tetra-O-acetyl-2-[¹⁸F]fluor-2-desoxy-D-glucose eingesetzt wird.

8. Verfahren nach einem der Ansprüche 1 bis 7,
**dadurch gekennzeichnet,**
**daß** die Verbindung nach Formel 1 trägerfrei ist.

9. Glycosylierungsreagenz,
**dadurch gekennzeichnet,**
**daß** es eine Struktur der allgemeinen Formel 2 hat, 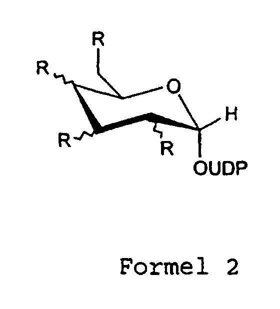 bei welcher der Hexoserest in den Postionen 2,3,4 und 6 Reste R besitzt, von denen drei Reste R eine OH-Gruppe und ein Rest R ein ¹⁸F-Atom bedeuten, wobei sich das ¹⁸F-Atom in der Position 2,3,4 oder 6 des Hexoserings befindet.

10. Glycosylierungsreagenz nach Anspruch 9,
**dadurch gekennzeichnet,**
**daß** sich das ¹⁸F-Atom des Hexoserings in Position 2 befindet.

11. Verwendung eines Glycosylierungsreagenz nach einem der Ansprüche 9 oder 10 für die ¹⁸F-Markierung von glykosylierten Peptiden, glykosylierten Proteinen und Oligosacchariden sowie Sacchariden.

## Claims

1. Process for the manufacture of an^{18F}-labelled glycosylation reagent,
**characterised in that** a hexose of the general Formula 1 with 4 residues R 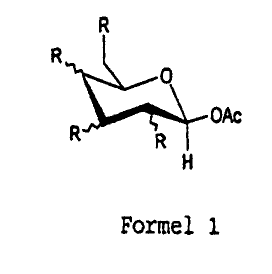
- where three of the four residues serve as a protective group which is bound via oxygen atoms to the hexagonal ring,
- and a residue R means ¹⁸F, where R = ¹⁸F is in position 2, 3, 4 or 6 of the hexose,
1. is caused to react to H₃PO₄,
2. subsequently hydrolised with a base, and
3. thereafter a reaction is caused by adding UTP by means of a pyrophosphorylase in the presence of Mg²⁺.

2. Process according to Claim 1,
**characterised in that**
a hexose of the general Formula 1 is applied, the protective group R of which is an OAc group.

3. Process according to one of Claims 1 or 2,
**characterised in that**
the reaction according to Step 1 with H₃PO₄ is performed by fusion.

4. Process according to one of Claims 1 to 3,
**characterised in that**
the hydrolysis with a base is carried out according to Step 2 by means of LiOH.

5. Process according to one of Claims 1 to 4,
**characterised in that**
in Step 3 a uridinediphosphateglucose pyrophosphorylasis (EC 2.7.7.9) is used.

6. Process according to Claim 6,
**characterised in that**
a uridinediphosphateglucose pyrophosphorylasis of beef liver or baking yeast is used.

7. Process according to one of Claims 1 to 6,
**characterised in that** as compound according to Formula 1 is used 1.3.4.6-tetra-O-acetyl-2-[¹⁸F]fluor-2-desoxy-D-glucose.

8. Process according to one of Claims 1 to 7,
**characterised in that**
the compound according to Formula 1 is unsupported.

9. Glycosylation reagent,
**characterised in that**
it has a structure of general Formula 2 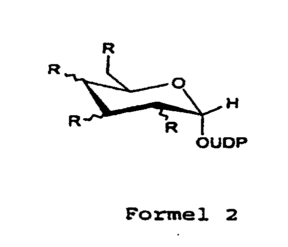 where the hexose residue in positions 2, 3, 4 and 6 includes residues R of which three residues R are an OH group and one residue an ¹⁸F atom, and the ¹⁸F-atom is in position 2, 3, 4 or 6 of the hexose ring.

10. Glycosylation reagent according to Claim 9,
**characterised in that**
the ¹⁸F-atom of the hexose ring is in position 2.

11. Use of a glycosylation reagent according to one of Claims 9 or 10 for ¹⁸F-labelling of glycosylated peptides, glycosylated proteins and oligosaccharides as well as saccharides.

## Revendications

1. Procédé d'obtention d'un réactif de glycosylation marqué au fluor¹⁸, **caractérisé en ce qu'**un hexose de formule générale 1 avec 4 résidus R : 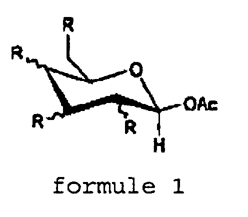
- dans lequel trois des quatre résidus désignent un groupe protecteur, liés au moyen d'un atome d'oxygène au niveau d'un noyau à six chaînons,
- et un résidu R désigne du fluor¹⁸, lequel R = fluor¹⁸ se trouve en position 2, 3, 4 ou 6 de l'hexose,
1. est converti avec du H₃PO₄,
2. est ensuite hydrolysé avec une base,
3. et ensuite, est converti par l'ajout de UTP au moyen d'une pyrophosphorylase en présence de Mg²⁺.

2. Procédé selon la revendication 1, **caractérisé en ce qu'**un hexose de formule générale 1 est utilisé, dont le groupe de protection R est un groupe OAc.

3. Procédé selon l'une quelconque des revendications 1 à 2, **caractérisé en ce que** la conversion selon l'étape 1 avec du H₃PO₄ est réalisée au moyen de la fusion.

4. Procédé selon l'une quelconque des revendications 1 à 3, **caractérisé en ce que** l'hydrolyse avec une base selon l'étape 2 est réalisée au moyen de LiOH.

5. Procédé selon l'une quelconque des étapes 1 à 4, **caractérisé en ce qu'**au cours de l'étape 3, une uridine diphosphoglucose pyrophosphorylase (EC 2.7.7.9) est utilisée.

6. Procédé selon la revendication 6, **caractérisé en ce qu'**une uridine diphosphoglucose pyrophosphorylase est obtenue à partir du foie de veau ou à partir de la levure de boulanger.

7. Procédé selon l'une quelconque des revendications 1 à 6, **caractérisé en ce que** l'on utilise comme composé selon la formule 1 le 1,3,4,6-tétra-O-acétyl-2-[¹⁸F]fluoro-2-désoxy-D-glucose.

8. Procédé selon l'une quelconque des revendications 1 à 7, **caractérisé en ce que** le composé de formule 1 est libre de tout support.

9. Réactif de glycosylation, **caractérisé en ce qu'**il possède une structure de formule générale 2, 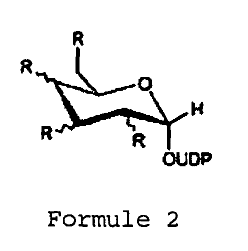 dans laquelle le résidu d'hexose possède en positions 2, 3, 4 et 6 des résidus R, parmi lesquels trois résidus R désignent un groupe OH et un résidu R désigne un atome de fluor¹⁸, l'atome de fluor¹⁸ se trouvant en positions 2, 3, 4 ou 6 du noyau d'hexose.

10. Réactif de glycosylation selon la revendication 9, **caractérisé en ce que** l'atome de fluor¹⁸ du noyau d'hexose se trouve en position 2.

11. Utilisation d'un réactif de glycosylation selon l'une quelconque des revendications 9 ou 10 pour le marquage au fluor¹⁸ des peptides glycosylés, des protéines glycosylées ou des oligosaccharides ainsi que des saccharides.
